# EUROPÄISCHE PATENTSCHRIFT

(11) **EP 1 124 781 B1**
(45) Veröffentlichungstag und Bekanntmachung des Hinweises auf die Patenterteilung: **11.06.2003**
(21) Anmeldenummer: 99953827.5
(22) Anmeldetag: 14.10.1999
(51) Int. Cl.: C07C 47/04, C07C 45/78, C07C 45/86, C07C 29/42, C07C 29/17, C07C 33/046, C07C 31/20

(54) **TETROXANARME WÄSSERIGE FORMALDEHYDLÖSUNGEN**
AQUEOUS FORMALDEHYDE SOLUTIONS WITH A LOW TETROXANE CONTENT
SOLUTIONS AQUEUSES DE FORMALDEHYDE A FAIBLE TENEUR EN TETROXANE

(30) Priorität: 27.10.1998 DE 19849380
(43) Veröffentlichungstag der Anmeldung: 22.08.2001
(73) Patentinhaber: BASF AKTIENGESELLSCHAFT, 67056 Ludwigshafen (DE)
(72) Erfinder: DANZ, Eckehard, D-67071 Ludwigshafen (DE); PANDL, Klaus, D-76707 Hambrücken (DE)
(86) Internationale Anmeldenummer: EP9907744
(87) Internationale Veröffentlichungsnummer: WO00024699

(56) Entgegenhaltungen:
- DE-C- 854 502
- GB-A- 931 892
- GB-A- 1 037 934
- GB-A- 2 023 020
- US-A- 1 481 849
- US-A- 4 584 418
- CHEMICAL ABSTRACTS, vol. 088, no. 17, 24. April 1978 (1978-04-24) Columbus, Ohio, US; abstract no. 120611, KAMIYAMA T ET AL: "Stabilization of mixtures containing highly concentrated formaldehyde" XP002128204 & JP 52 153905 - (MATSUSHITA ELECTRIC WORKS, LTD.;JAPAN)

## Beschreibung

Die Erfindung betrifft tetroxanarme wässerige Formaldehydlösungen mit einem pH-Wert von 3,4 bis 4,2, enthaltend
a) 10 bis 75 Gew.-% Formaldehyd
b) 0 bis 500 Gew.-ppm Tetroxan

Weiterhin betrifft die Erfindung Verfahren zu deren Herstellung sowie ein Verfahren zur Herstellung von 1,4-Butandiol durch Umsetzung der tetroxanarmen wässerigen Formaldehydlösungen mit Acetylen zu 1,4-Butindiol und anschließende Hydrierung des 1,4-Butindiols zu 1,4-Butandiol.

Wässerige Formaldehydlösungen und Verfahren zu deren Herstellung sind allgemein bekannt (vgl. Ullmanns Encyclopädie der technischen Chemie, Verlag Chemie, GmbH, Weinheim, 4. Auflage, Band 11, Stichwort Formaldehyd, Kapitel 3).

Hierzu wird im allgemeinen zunächst Methanol mit Luft oxidativ dehydrogeniert und der dabei gebildete formaldehydhaltige Gasstrom in einer Absorptionszone mit Wasser in Kontakt gebracht, wobei eine wässerige Formaldehydlösung gebildet wird.

Die dabei gebildeten Formaldehydiösungen weisen jedoch den Nachteil auf, daß sich das Formaldehyd bereits innerhalb kürzester Zeit teilweise zu Tetroxan der Formel (I) 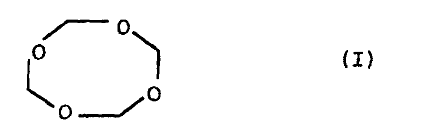 umsetzt, so daß sich in konzentrierten Formaldehydlösungen häufig bereits innerhalb eines Tages nach deren Herstellung einige hundert ppm Tetroxan bilden.

Formaldehydlösungen werden als Zwischenprodukt bei der Synthese vieler chemischer Substanzen eingesetzt. In vielen Fällen wird dabei das Tetroxan nicht umgesetzt sondern verbleibt bei der Aufarbeitung der Reaktionsmischung als Rückstand im Abwasser. Da Tetroxan nach einschlägigen Umweltrichtlinien als wassergefährdend eingestuft wird, müssen derartige Abwässer durch aufwendige Reinigungsoperationen vom Tetroxan befreit werden. Diese Problematik zeigt sich insbesondere bei der Herstellung von 1,4-Butandiol durch Umsetzung der wässerigen Formaldehydlösungen mit Acetylen zu 1,4-Butindiol und anschließende Hydrierung des 1,4-Butindiols zu 1,4-Butandiol, wobei das Tetroxan in einer Mischung mit Wasser und dem Wertprodukt 1,4-Butandiol anfällt.

Die Aufgabe der vorliegenden Erfindung bestand deshalb darin, wässerige Formaldehydlösungen bereitzustellen, die trotz längerer Lagerung kein oder nur geringe Mengen an Tetroxan enthalten.

Demgemäß wurden die eingangs beschriebenen wässerigen Lösungen, Verfahren zu deren Herstellung sowie ein Verfahren zur Herstellung von 1,4-Butandiol unter Verwendung dieser Lösungen bereitgestellt.

Da Formaldehydlösungen im allgemeinen als Lösungen mit einem Formaldehydgehalt von 30 bis 60 Gew.-% gehandelt werden, enthalten die bevorzugten erfindungsgemäßen wässerigen Lösungen üblicherweise ebenfalls Formaldehyd in diesen Konzentrationen.

Die erfindungsgemäßen wässerigen Lösungen enthalten in der Regel 50 bis 400, bevorzugt 50 bis 300 Gew.-ppm Ameisensäure und 0,2 bis 5,0, bevorzugt 0,5 bis 2,5 Gew.-% Methanol, da bei den meisten großtechnischen Verfahren zur Herstellung von Formaldehyd das Formaldehyd in Form von mit Formaldehyd beladenen Gasströmen gebildet wird, die üblicherweise
- 5 bis 30 Gew.-% Formaldehyd
- 0,5 bis 2,0 Gew.-% Methanol
- 30 bis 55 Gew.-% Stickstoff
und gegebenenfalls Spuren von Ameisenssäure enthalten.

Die großtechnische Herstellung von Formaldehyd und wässerigen Formaldehyd-Lösungen ist allgemein bekannt und beispielsweise in Ullmanns Encyclopädie der technischen Chemie, Verlag Chemie, GmbH, Weinheim, 4. Auflage, Band 11, Stichwort Formaldehyd, Kapitel 3 beschrieben.

Bei der anschließenden Herstellung von wässerigen Formaldehydlösungen durch selektive Absorption dieser mit Formaldehyd beladenen Gasströme in Wasser läßt es sich nicht vermeiden, daß ein gewisser Teil Methanol und Ameisensäure mit absorbiert wird. Weiterhin wird bei der Lagerung der Lösungen Methanol und Ameisensäure durch Disproportionierung von Formaldehyd gebildet.

Die Bildung von Tetroxan in diesen Lösungen läßt sich wirksam vermeiden oder verlangsamen, indem man ihren pH-Wert auf 3,4 bis 4,2 einstellt.

Dies geschieht mit Hilfe von Brönsted-Basen wie Ammoniak, ein Alkalihydroxid oder ein Alkalicarbonat. Im allgemeinen enthalten die wässerigen Formaldehyd-Lösungen 0,001 bis 0,1 Gew.-% der Brönsted-Base, bezogen auf den darin absorbierten Formaldehyd.

Günstigerweise geht man dabei so vor, dass man
I. einen mit Formaldehyd beladenen Gasstrom in Wasser absorbiert und
II. innerhalb von 30 min. nach der Absorption 0,001 bis 0,1 Gew.-% einer Brönsted-Base, bezogen auf den absorbierten Formaldehyd, löst.

Besonders günstig geht man zur Herstellung der erfindungsgemäßen Lösungen vor, daß man die Base bereits unmittelbar nach oder während der Herstellung der konventionellen Formaldehydlösungen zusetzt. Dies kann beispielsweise großtechnisch erfolgen, indem man ein Reaktionsgas, wie es bei der oxidativen Dehydrogenierung von Methanol mittels Luft an einem Silberkatalysator anfällt, in mehreren Absorptionsstufen in Wasser bzw. wässerigen Formaldehydlösungen absorbiert und der Absorptionsstufe, der das Reaktionsgas direkt zugeführt wird, die Base zusetzt.

Nach dieser Variante stellt man die erfindungsgemäßen Formaldehydlösungen also her, indem man:
in einer 1. Absorptionsstufe
- einen Formaldehyd-teilentladenen Gasstrom I (1) in einer mit Formaldehyd-teilbeladenen wässerige Lösung I (2) teilweise absorbiert, wobei eine Formaldehyd-teilbeladene wässerige Lösung II (3) und ein Formaldehyd-entladener Gasstrom II (4) mit verringertem Formaldehyd-Anteil gebildet wird, indem man den Formaldehyd-teilentladenen Gasstrom I (1) und die Formaldehyd-teilbeladene wässerige Lösung I (2) im Gegenstrom durch eine Absorptionszone I (5) führt,
- den Formaldehyd-entladenen Gasstrom II (4) und die Formaldehyd-teilbeladene wässerige Lösung II (3) aus der Extraktionszone I (5) entfernt,
- die Formaldehyd-teilbeladene wässerige Lösung II (3) in 2 Teilströme I (6) und II (7) teilt,
- den Teilstrom I (6) zusammen mit einer wässerigen Lösung W 5 (8), die kein Formaldehyd oder geringere Mengen davon enthält als die Formaldehyd-teilbeladene wässerige Lösung I (2), in die Absorptionszone I (5) leitet, wobei Teilstrom I (6) und die wässerige Lösung W gemeinsam die Formaldehyd-teilbeladene wässerige Lösung I (2) bilden
und in einer weiteren vorgeschalteten Absorptionsstufe 2
- einen Formaldehyd-beladenen Gasstrom III (9) in einer Formaldehyd-teilbeladenen wässerige Lösung III (10) teilweise oder ganz absorbiert, wobei eine Formaldehyd-beladene wässerige Lösung IV (11), bei der es sich um das gewünschte Wertprodukt handelt, und der Formaldehyd-teilentladene Gasstrom I (1) gebildet wird, indem man den Formaldehyd-beladenen Gasstrom III (9) und die Formaldehyd-teilbeladene wässerige Lösung III (10) im Gegenstrom durch eine Absorptionszone II (12) führt,
- die Formaldehyd-beladene wässerige Lösung IV (11) und den Formaldehyd-teilentladenen Gasstrom I (1) aus der Absorptionszone II (12) entfernt,
- die Formaldehyd-beladene wässerige Lösung IV (11) in 2 Teilströme III (13) und IV (14) teilt,
- einen Teilstrom V (15) herstellt, indem man dem Teilstrom III (13) pro Zeiteinheit und pro Gewichtsteil Formaldehyd, den man in Form des Formaldehyd-beladenen Gasstroms III (9) in die Absorptionszone II (12) leitet, 0,001 bis 0,01 Gew.-% einer Brönsted-Base (16) zusetzt und
- den Teilstrom V (15) zusammen mit Teilstrom II (7) zusammen in die Absorptionszone II leitet, wobei Teilstrom V (15) und II (7) gemeinsam die Formaldehyd-teilbeladene wässerige Lösung III (10) bilden.

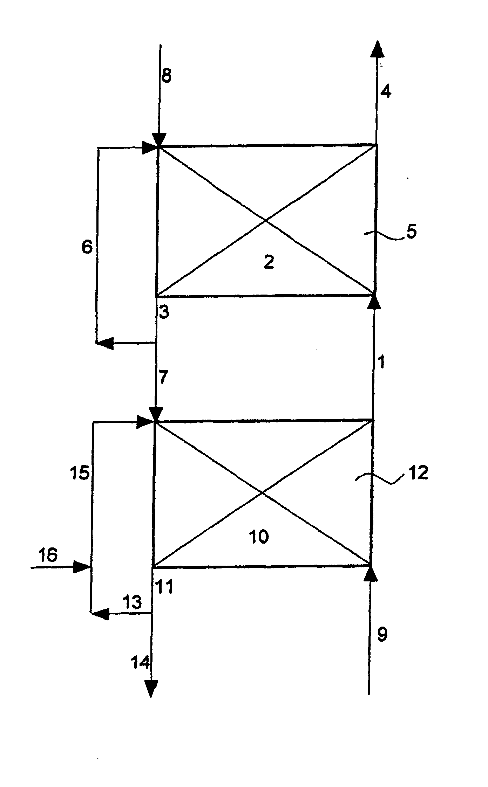

Diese Vorgehensweise wird in Figur 1 schematisch dargestellt.

Bei dieser Verfahrensweise kommen insbesondere solche Formaldehyd-beladenen Gasströme 3 in Betracht, wie sie bei der großtechnischen Herstellung von Formaldehyd gebildet werden.

Günstigerweise schaltet man 2 oder 3 Absorptionsstufen des Typs der Absorptionsstufe 1 hintereinander und setzt in der letzten Absorptionsstufe als wässerige Lösung, die kein Formaldehyd oder geringere Mengen davon enthält als die Formaldehyd-teilbeladene wässerige Lösung I, Wasser ein.

Im allgemeinen sind die verschiedenen Absorptionsstufen in einem Absorptionsturm integriert.

Zweckmäßigerweise wird die Brönsted-Base dem Teilstrom III in Form einer verdünnten Lösung, bevorzugt in Form von verdünnter Natronlauge zugesetzt.

Die auf diese Weise hergestellten Lösungen enthalten bevorzugt 10 bis 50 Gew.-ppm Formaldehyd.

Sie weisen den Vorteil auf, daß in ihnen die Bildung von Tetroxan nur sehr langsam erfolgt.

Der geringe Tetroxangehalt wirkt sich besonders positiv aus, wenn man diese Lösungen zur Herstellung von 1,4-Butandiol einsetzt. Hierbei geht man so vor, daß man die wässerigen Lösungen nach allgemein bekannten Verfahren mit Acetylen zu 1,4-Butindiol umsetzt und das 1,4-Butindiol anschließend zu 1,4-Butandiol hydriert.

### Beispiele

### Beispiel 1

100 ml einer wässerigen Formaldehydlösung enhaltend 49 Gew.-% Formaldehyd, 1,25 Gew.-% Methanol und 150 ppm Ameisensäure wurden ca. 2 ml einer 0,1 molaren Natronlauge zugesetzt und über mehrere Tage bei 60°C gelagert. Der Tetroxangehalt wurde täglich gemessen (Zeitpunkt der Zugabe der Natronlauge = Null-Tage-Wert)

### Vergleichsbeispiel

Es wurde wie in Beispiel 1 beschrieben vorgegangen, jedoch wurde der Lösung vor Beginn der Lagerung keine Natronlauge zugesetzt.

Das Ergebnis kann Tabelle 1 entnommen werden.

**Tabelle 1**

| Tage | Tetroxangehalt [ppm] | |
|---|---|---|
| | Beispiel 1 | Vergleichsbeispiel |
| 0 | 220 | 220 |
| 1 | 230 | 330 |
| 2 | 210 | 400 |
| 3 | 270 | 500 |

## Patentansprüche

1. Wässerige Lösung mit einem pH-Wert von 3,4 bis 4,2, enthaltend
a) 10 bis 75 Gew.-% Formaldehyd
b) 0 bis 500 Gew.-ppm Tetroxan

2. Wässerige Lösung nach Anspruch 1, enthaltend, bezogen auf den Formaldehyd
c) 50 bis 400 Gew.-ppm Ameisensäure und
d) 0,2 bis 5,0 Gew.-% Methanol

3. Verfahren zur Herstellung einer wässerigen Lösung nach Anspruch 1 oder 2, wobei man in einer Absorptionszone einen mit Formaldehyd beladenen Gasstrom in einer wässerigen Lösung, enthaltend 0,001 bis 0,01 Gew.-% einer Brönsted-Base, bezogen auf den absorbierten Formaldehyd, absorbiert.

4. Verfahren zur Herstellung einer wässerigen Lösung nach Anspruch 1 oder 2, wobei man
I. einen mit Formaldehyd beladenen Gasstrom in Wasser absorbiert und
II. innerhalb von 30 min nach der Absorption 0,001 bis 0,1 Gew.-% einer Brönsted-Base, bezogen auf den absorbierten Formaldehyd, löst.

5. Verfahren zur Herstellung einer wässerigen Lösung nach Anspruch 1 oder 2, wobei man kontinuierlich
in einer 1. Absorptionsstufe
- einen Formaldehyd-teilentladenen Gasstrom I in einer mit Formaldehyd-teilbeladenen wässerige Lösung I teilweise absorbiert, wobei eine Formaldehyd-teilbeladene wässerige Lösung II und ein Formaldehyd-entladener Gasstrom II mit verringertem Formaldehyd-Anteil gebildet wird, indem man den Formaldehyd-teilentladenen Gasstrom I und die Formaldehyd-teilbeladene wässerige Lösung I im Gegenstrom durch eine Absorptionszone I führt,
- den Formaldehyd-entladenen Gasstrom II und die Formaldehyd-teilbeladene wässerige Lösung II aus der Extraktionszone I entfernt,
- die Formaldehyd-teilbeladene wässerige Lösung II in 2 Teilströme I und II teilt,
- den Teilstrom I zusammen mit einer wässerigen Lösung W, die kein Formaldehyd oder geringere Mengen davon enthält als die Formaldehyd-teilbeladene wässerige Lösung I, in die Absorptionszone I leitet, wobei Teilstrom I und die wässerige Lösung W gemeinsam die Formaldehyd-teilbeladene wässerige Lösung I bilden
und in einer weiteren vorgeschalteten Absorptionsstufe 2
- einen Formaldehyd-beladenen Gasstrom III in einer Formaldehyd-teilbeladenen wässerige Lösung III teilweise absorbiert, wobei eine Formaldehyd-beladene wässerige Lösung IV, bei der es sich um die wässerige Lösung gemäß Anspruch 1 oder 2 handelt, und der Formaldehyd-teilentladene Gasstrom I gebildet wird, indem man den Formaldehyd-beladenen Gasstrom III und die Formaldehyd-teilbeladene wässerige Lösung III im Gegenstrom durch eine Absorptionszone II führt,
- die Formaldehyd-beladene wässerige Lösung IV und den Formaldehyd-teilentladenen Gasstrom I aus der Absorptionszone II entfernt,
- die Formaldehyd-beladene wässerige Lösung IV in 2 Teilströme III und IV teilt,
- einen Teilstrom V herstellt, indem man dem Teilstrom III pro Gewichtsteil Formaldehyd, den man in Form des Formaldehyd-beladenen Gasstroms III in die Absorptionszone II leitet, 0,001 bis 0,01 Gew.-% einer Brönsted-Base zusetzt und
- den Teilstrom V zusammen mit Teilstrom II zusammen in die Absorptionszone II leitet, wobei Teilstrom V und II gemeinsam die Formaldehyd-teilbeladene wässerige Lösung III bilden.

6. Verfahren nach Anspruch 5, **dadurch gekennzeichnet, daß** man 2 oder 3 Absorptionsstufen des Typs der ersten Absorptionsstufe hintereinanderschaltet und man bei der letzten Absorptionsstufe als wässerigen Lösung, die kein Formaldehyd oder geringere Mengen davon enthält als die Formaldehyd-teilbeladene wässerige Lösung I, Wasser einsetzt.

7. Verfahren nach den Ansprüchen 3 bis 6, wobei man als Brönsted- Base Ammoniak, ein Alkalihydroxid oder ein Alkalicarbonat einsetzt.

8. Verfahren nach den Ansprüchen 3 bis 7, wobei der mit Formaldehyd beladenen Gasstrom
- 5 bis 30 Gew.-% Formaldehyd
- 0,5 bis 2,0 Gew.-% Methanol
- 30 bis 55 Gew.-% Stickstoff
enthält.

9. Verfahren zur Herstellung von 1,4-Butandiol durch Umsetzung der wässerigen Lösungen nach den Ansprüchen 1 bis 3 mit Acetylen zu 1,4-Butindiol und anschließende Hydrierung des 1,4-Butindiols zu 1,4-Butandiol.

## Claims

1. An aqueous solution having a pH of from 3.4 to 4.2 comprising
a) from 10 to 75% by weight of formaldehyde
b) from 0 to 500 ppm by weight of tetroxane.

2. An aqueous solution as claimed in claim 1 comprising, based on the formaldehyde,
c) from 50 to 400 ppm by weight of formic acid and
d) from 0.2 to 5.0% by weight of methanol.

3. A process for preparing an aqueous solution as claimed in claim 1 or 2, a formaldehyde-loaded gas stream, in an absorption zone, being absorbed in an aqueous solution comprising from 0.001 to 0.01% by weight of a Brönsted base, based on the formaldehyde absorbed.

4. A process for preparing an aqueous solution as claimed in claim 1 or 2,
I. a formaldehyde-loaded gas stream being absorbed in water and
II. in the course of 30 min after the absorption, from 0.001 to 0.1% by weight of a Brönsted base, based on the formaldehyde absorbed, being dissolved.

5. A process for preparing an aqueous solution as claimed in claim 1 or 2, with, continuously,
in a 1st absorption stage
- a part-formaldehyde-depleted gas stream I being partially absorbed in a part-formaldehyde-loaded aqueous solution I, a part-formaldehyde-loaded aqueous solution II and a formaldehyde-depleted gas stream II having decreased formaldehyde content being formed, by conducting the part-formaldehyde-depleted gas stream I and the part-formaldehyde-loaded aqueous solution I in countercurrent through an absorption zone I,
- the formaldehyde-depleted gas stream II and the part-formaldehyde-loaded aqueous solution II being removed from the extraction zone I,
- the part-formaldehyde-loaded aqueous solution II being divided into 2 partial streams I and II,
- the partial stream I being passed into the absorption zone I together with an aqueous solution W which comprises no formaldehyde or smaller amounts thereof than the part-formaldehyde-loaded aqueous solution I, partial stream I and the aqueous solution W together forming the part-formaldehyde-loaded aqueous solution I
and in a further upstream absorption stage 2
- a formaldehyde-loaded gas stream III being partially absorbed in a part-formaldehyde-loaded aqueous solution III, a formaldehyde-loaded aqueous solution IV, which solution IV is the aqueous solution as claimed in claim 1 or 2, and the part-formaldehyde-depleted gas stream I being formed, by conducting the formaldehyde-loaded gas stream III and the part-formaldehyde-loaded aqueous solution III in countercurrent through an absorption zone II,
- the formaldehyde-loaded aqueous solution IV and the part-formaldehyde-depleted gas stream I being removed from the absorption zone II,
- the formaldehyde-loaded aqueous solution IV being divided into 2 partial streams III and IV,
- a partial stream V being produced by adding from 0.001 to 0.01% by weight of a Brönsted base to the partial stream III per part by weight of formaldehyde which is passed into the absorption zone II in the form of the formaldehyde-loaded gas stream III and
- the partial stream V being passed together with partial stream II together into the absorption zone II, partial stream V and II together forming the part-formaldehyde-loaded aqueous solution III.

6. A process as claimed in claim 5, wherein 2 or 3 absorption stages of the first absorption stage type are connected in series and, in the last absorption stage, as aqueous solution which comprises no formaldehyde or smaller amounts thereof than the part-formaldehyde-loaded aqueous solution I, use is made of water.

7. A process as claimed in claims 3 to 6, the Brönsted base used being ammonia, an alkali metal hydroxide or an alkali metal carbonate.

8. A process as claimed in claims 3 to 7, the formaldehyde-loaded gas stream comprising
- from 5 to 30% by weight of formaldehyde
- from 0.5 to 2.0% by weight of methanol
- from 30 to 55% by weight of nitrogen.

9. A process for preparing 1,4-butanediol by reacting the aqueous solutions as claimed in claims 1 to 3 with acetylene to form 1,4-butynediol and subsequent hydrogenation of the 1,4-butynediol to form 1,4-butanediol.

## Revendications

1. Solution aqueuse ayant une valeur de pH de 3,4 à 4,2 contenant
a) 10 à 75% en poids de formaldéhyde
b) 0 à 500 ppm en poids de tétroxane

2. Solution aqueuse selon la revendication 1 contenant, par rapport au formaldéhyde,
c) 50 à 400 ppm en poids d'acide formique et
d) 0,2 à 5,0% en poids de méthanol.

3. Procédé de préparation d'une solution aqueuse selon la revendication 1 ou 2, dans lequel on absorbe un courant gazeux chargé en formaldéhyde dans une solution aqueuse, contenant 0,001 à 0,01% en poids d'une base de Brönsted, par rapport au formaldéhyde absorbé, dans une zone d'absorption.

4. Procédé de préparation d'une solution aqueuse selon la revendication 1 ou 2, dans lequel
I. on absorbe un courant gazeux chargé en formaldéhyde dans de l'eau, et
II. on dissout en l'espace de 30 min après l'absorption, 0,001 à 0,1% en poids d'une base de Brönsted, par rapport au formaldéhyde absorbé.

5. Procédé de préparation d'une solution aqueuse selon la revendication 1 ou 2, dans lequel
dans un 1er étage d'absorption
- on absorbe partiellement un courant gazeux I partiellement déchargé en formaldéhyde dans une solution aqueuse I partiellement chargée en formaldéhyde, une solution aqueuse II partiellement chargée en formaldéhyde et un courant gazeux II chargé en formaldéhyde avec une teneur en formaldéhyde réduite étant formés, en conduisant le courant gazeux I partiellement déchargé en formaldéhyde et la solution aqueuse I partiellement chargée en formaldéhyde à contre-courant à travers une zone d'absorption I,
- on retire le courant gazeux II déchargé en formaldéhyde et la solution aqueuse II partiellement chargée en formaldéhyde de la zone d'extraction I,
- on partage la solution aqueuse II partiellement chargée en formaldéhyde en 2 courants partiels I et II,
- on conduit le courant partiel I conjointement avec une solution aqueuse W, qui ne contient pas de formaldéhyde ou bien qui n'en contient que des quantités réduites, en tant que solution aqueuse I partiellement chargée en formaldéhyde, dans la zone d'absorption I, le courant partiel I et la solution aqueuse W formant conjointement la solution aqueuse I partiellement chargée en formaldéhyde,
et dans un étage d'absorption supplémentaire 2 en amont
- on absorbe partiellement un courant gazeux III chargé en formaldéhyde dans une solution aqueuse III partiellement chargée en formaldéhyde, une solution aqueuse IV chargée en formaldéhyde, s'agissant de la solution aqueuse selon la revendication 1 ou 2, et le courant gazeux I partiellement déchargé en formaldéhyde étant formés, en conduisant le courant gazeux III chargé en formaldéhyde et la solution aqueuse III partiellement chargée en formaldéhyde à contre-courant à travers une zone d'absorption II,
- on retire la solution aqueuse IV chargée en formaldéhyde et le courant gazeux I partiellement déchargé en formaldéhyde de la zone d'absorption II,
- on partage la solution aqueuse IV chargée en formaldéhyde en deux courants partiels III et IV,
- on prépare un courant partiel V en ajoutant au courant partiel III, 0,0001 à 0,01% en poids d'une base de Brönsted, par partie en poids de formaldéhyde, que l'on conduit sous la forme du courant gazeux III chargé en formaldéhyde dans la zone d'absorption II, et
- on conduit le courant partiel V conjointement avec le courant partiel II conjointement dans la zone d'absorption II, le courant partiel V et II formant conjointement la solution aqueuse III partiellement chargée en formaldéhyde.

6. Procédé selon la revendication 5, **caractérisé en ce que** l'on monte l'un derrière l'autre, 2 ou 3 étages d'absorption du type du premier étage d'absorption, et que dans le dernier étage d'absorption, l'on met en oeuvre de l'eau sous forme de solution aqueuse qui ne contient pas de formaldéhyde ou bien qui n'en contient que des quantités réduites, en tant que solution aqueuse I chargée partiellement en formaldéhyde.

7. Procédé selon les revendications 3 à 6, dans lequel on met en oeuvre en tant que base de Brönsted, l'ammoniac, un hydroxyde de métal alcalin ou un carbonate de métal alcalin.

8. Procédé selon les revendications 3 à 7, dans lequel le courant gazeux chargé en formaldéhyde contient
- 5 à 30% en poids de formaldéhyde
- 0,5 à 2,0% en poids de méthanol
- 30 à 55% en poids d'azote.

9. Procédé de préparation du 1,4-butanediol par conversion des solutions aqueuses selon les revendications 1 à 3, avec l'acétylène en 1,4-butynediol suivie par l'hydrogénation du 1,4-butynediol en 1,4-butanediol.
